# EUROPÄISCHE PATENTANMELDUNG

(11) **EP 1 110 572 A1**
(43) Veröffentlichungstag der Anmeldung: **27.06.2001**
(21) Anmeldenummer: 99125842.7
(22) Anmeldetag: 24.12.1999
(51) Int. Cl.: A61M 15/00

(54) **Inhalationsgerät**

(71) Anmelder: Kölbel, Gert F., 30900 Wedemark (DE)
(72) Erfinder: Kölbel, Gert F., 30900 Wedemark (DE)
(74) Vertreter: Arendt, Helmut, Dipl.-Ing.

(57) **Zusammenfassung**

Zum Inhalieren von Heilstoffen wird ein nicht ortsgebundenes Gerät vorgeschlagen, das vom Patienten auch während der Ausübung weiterer Tätigkeiten benutzt werden kann und aus einem Gehäuse mit zwei ständigen Öffnungen besteht, in welchem sich ein Wirkstoffträger befindet. Eine der Öffnungen ist für das Ansaugen von Frischluft vorgesehen. Die zweite Öffnung dient zum Inhalieren und ist mit einem Mundstück zum Halten des Gerätes mit dem Mund ausgerüstet.

## Beschreibung

Die Erfindung betrifft ein Gerät zum Inhalieren von Heilstoffen.

Das Einatmen oder Inhalieren von gasförmigen oder feinstzerstäubten, flüssigen Heil- oder Arzneistoffen ist eine bewährte Behandlungsmethode für verschiedene Krankheiten des Nasen-Rachenraumes und der tieferen Luftwege. Bekannt sind das Einatmen von heißen, mit Heilmitteln angereicherten Dämpfen und zerstäubten Flüssigkeiten mit Hilfe eines elektrisch betriebenen Gerätes. Angestrebt wird eine möglichst feine Auflösung des Inhalats, da mit einem sehr feinen Auflösungsgrad die in Schwebetröpfchen enthaltenen Wirkstoffe tiefer in die Luftwege und bis in die kleinsten Bronchienäste eindringen können.

Das Inhalieren der in den Tröpfchen enthaltenen Wirkstoffe erfolgt dabei ungesteuert, so daß der Atemstrom zwangsweise die Menge an Wirkstoffen aufnimmt, die gerade von einem Zerstäuber mitgerissen werden oder im heißen Dampf enthalten sind. Eine während der Inhalierdauer gesteuerte oder dosierte Einbringung der Wirkstoffe in den Atemstrom ist nicht bekannt.

Inhaliergeräte, auch Tischgeräte, sind von erheblicher Baugröße. Die Mundstücke oder Atemmasken werden von Hand an den Mund gepreßt. Bekannt ist auch das Auflegen des Gesichts in vorgebeugter Haltung bei Tischgeräten auf die Inhaliermaske.

Der Inhalierende ist während der Dauer der Inhalation ortsgebunden und davon abgehalten, anderen Tätigkeiten nachzugehen. Wegen der teilweise umständlichen Handhabung der Geräte ist es nicht möglich, Dauerinhalationen über mehrere Stunden oder gar über Nacht durchzuführen, was aber von der Indikation her wünschenswert wäre.

Der Erfindung liegt die Aufgabe zugrunde, ein Gerät zum Inhalieren zu schaffen, das nicht ortsgebunden ist und vom Patienten über mehrere Stunden benutzt werden kann, ohne daß deswegen jede Tätigkeit oder Arbeit unterbrochen werden muß. Die erfindungsgemäße Lösung zeichnet sich dadurch aus, daß in einem Gehäuse mit zwei ständigen öffnungen ein Wirkstoffträger angeordnet ist, wobei eine der Öffnungen für den Eintritt von angesaugter Frischluft vorgesehen ist und die zweite Öffnung als Inhalieröffnung dient und mit einem Mundstück zum unmittelbaren Halten des Gerätes mit dem Mund ausgerüstet ist.

Das erfindungsgemäße Kleingerät vermeidet die vorgenannten Nachteile bekannter Ausführungen und läßt sich wesentlich einfacher und damit preiswerter herstellen und anbieten. Die Dosierung des Inhalats läßt sich beispielsweise durch eine teilweise Abdeckung des Wirkstoffträgers steuern. Der Transport der Wirkstoffe wird allein durch die Atemluft besorgt, der sich durch Verdunstung die Wirkstoffe beimischen. Dadurch kann der Wirkstoff in wesentlich feinerer Verteilung in der Atemluft angeboten werden, so daß er ohne Schwierigkeiten bis in die feinsten Verästelungen der Bronchien dringen kann. Gleichzeitig wird die Wirkung der angebotenen Arzneimittel verbessert.

Das Betreiben des Gerätes durch Fremdenergie enfällt. Es ist nicht an das Vorhandensein einer elektrischen Stromquelle gebunden. Die höhere Temperatur des ausgeatmeten Luftstromes wärmt ätherische öle für die darauffolgende Einatmung vor und sorgt für eine bessere Bereitstellung von Wirkstoffen. Gleichzeitig entfällt eine übertemperierung. Nicht jeder Wirkstoff kann bei Temperaturen weit oberhalb der Körpertemperatur seine heilende Eigenschaft entfalten oder aufrechterhalten.

Das erfindungsgemäße Inhaliergerät ist mit einem Mundstück ausgerüstet, das zwischen der vorderen Zahnreihe und den davor liegenden Lippen gehalten wird. Auch bei entspanrter Lippenmuskulatur, z. B. während des Schlafes, ist ein guter Sitz des Gerätes gewährleistet. Der Atemstrom wird bei seinem ausschließlichen Weg durch das erfindungsgemäße Gerät nicht einem unnötigen Widerstand ausgesetzt. Die öffnungsquerschnitte für die Atemluftdurchströmung können so gewählt werden, daß selbst bei verstärktem Atemluftbedarf, beispielsweise bei leichten Sportübungen, eine uneingeschränkte Sauerstoffversorgung stattfinden kann.

In weiterer vorteilhafter Gestaltung des Gerätes können zusätzlich zwei weitere öffnungen zum Aufstecken von Nasenröhrchen vorgesehen werden, um den Atemstrom nicht nur durch den Mundraum, sondern auch durch den Nasenraum zu führen.

Während der Inhalation bleiben beide Hände frei und der Patient urgebunden. Er kann während der Inhalation seinem Beruf nachgehen, sich mit seinem Fahrrad oder seinem Kraftfahrzeug fortbewegen, schlafen und sogar sprechen.

Außerhalb der Heilbehandlung kann der erfindungsgemäße Inhalator einen weiteren Anwendungsbereich finden. In bakteriell belasteter oder ungesunder Luft ist es möglich, dem Anwender durch die Verwendung bestimmter ätherischer öle oder anderer Wirkstoffe im Gerät die Atemluft gesünder, beispielsweise keimfrei zuzuführen.

Mit einer einseitig offenen Abdeckhülse kann der Wirkstoffträger völlig verschlossen und bei Gebrauch des Gerätes kontinuierlich bis zur völligen Freigabe der Atemluft ausgesetzt werden. Dadurch ist eine besonders einfache, jedoch wirksame Dosierung des Wirkstoffs im Atemstrom erreichbar. Bei völlig geschlossenem Wirkstoffträger wird die unverbrauchte Wirkstoffmenge im Gerät aufbewahrt und kann für eine spätere Verwendung mitgeführt werden.

Durch die dauerhafte Inhalation auch während der Nachtzeit ist es insbesondere für Schwerkranke, z. B. Asthmatiker, möglich, die Anfallbereitschaft ständig zu unterdrücken und nicht nur für kurze Zeitintervalle.

In der Zeichnung ist ein Ausführungsbeispiel der Erfindung dargestellt und nachstehend erläutert. Es zeigen:

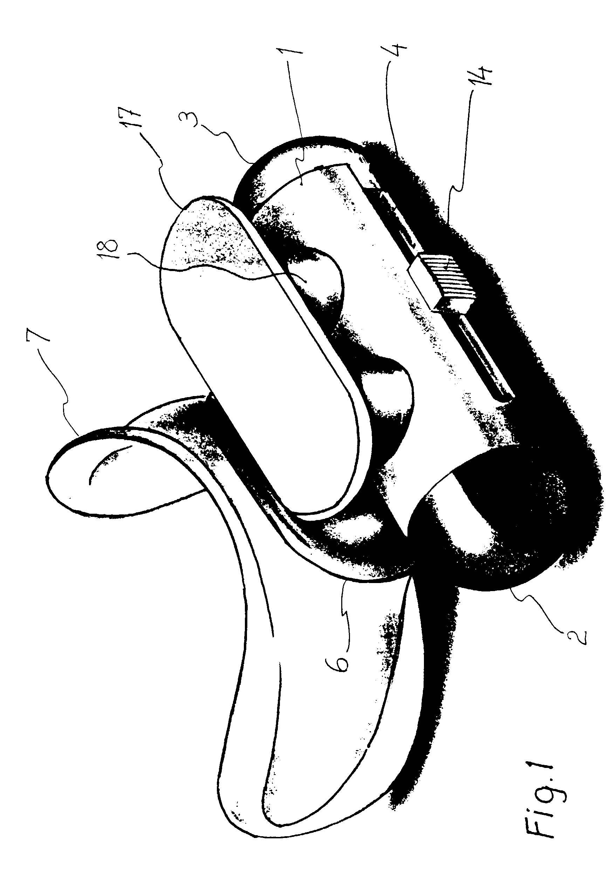

- Figur 1: die schaubildliche Darstellung des Gerätes mit verschlossenen Nasenöffnungen,

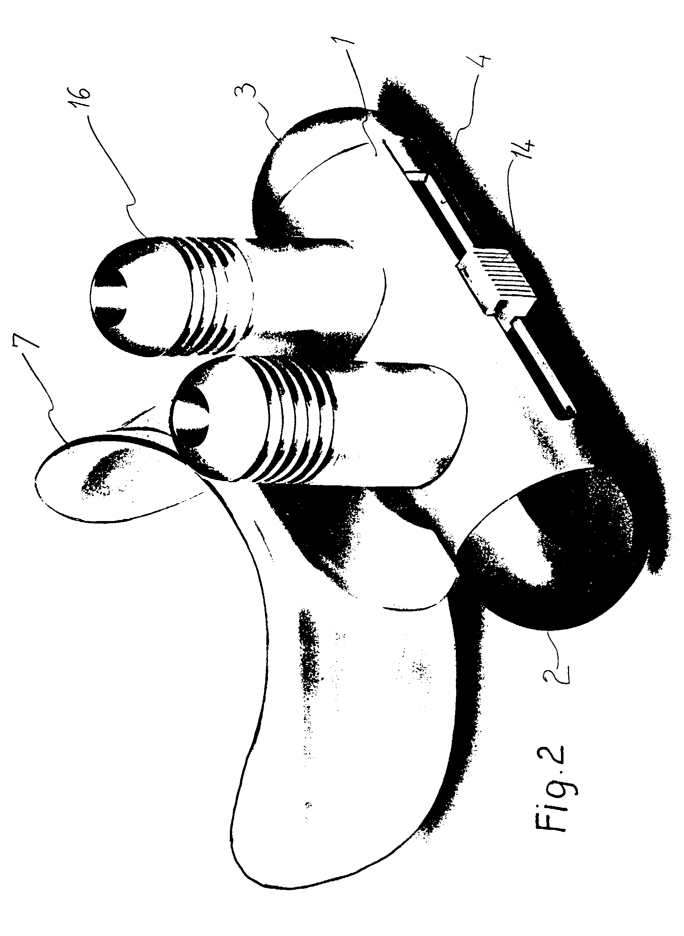

- Figur 2: das Gerät mit aufgesteckten Nasenröhrchen zur Inhalation durch die Nasenöffnungen,

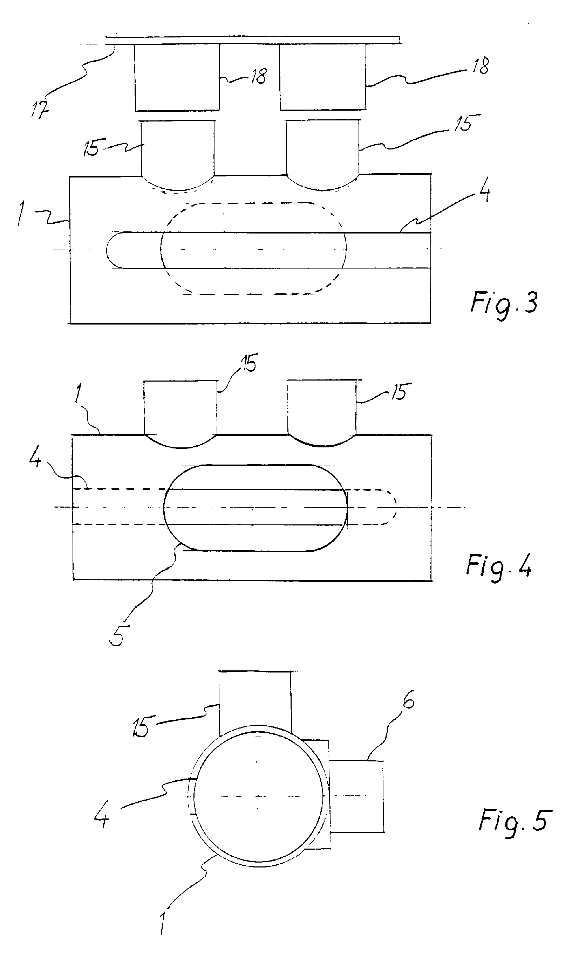

- Figur 3: eine Seitenansicht des hülsenförmigen Mittelteils,
- Figur 4: das Mittelteil von der rückwärtigen Ansicht mit der Anschlußöffnung für das Mundstück,
- Figur 5: eine Stirnansicht des Mittelteils,

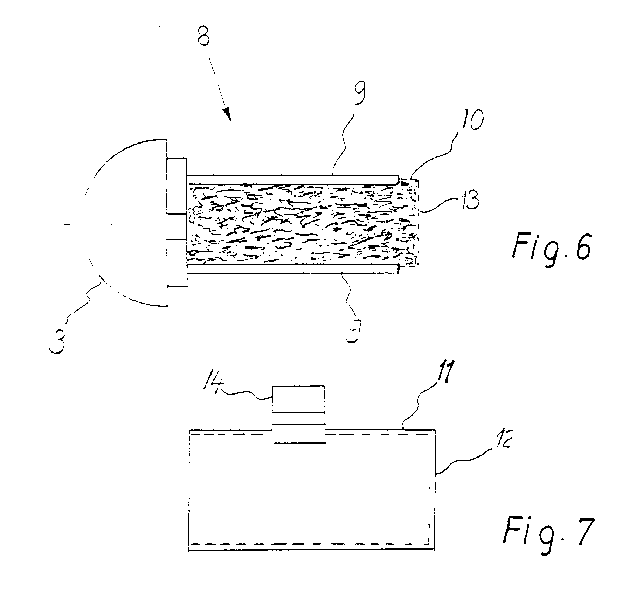

- Figur 6: die Seitenansicht des Wirkstoffhalters,
- Figur 7: die Dosierhülse,

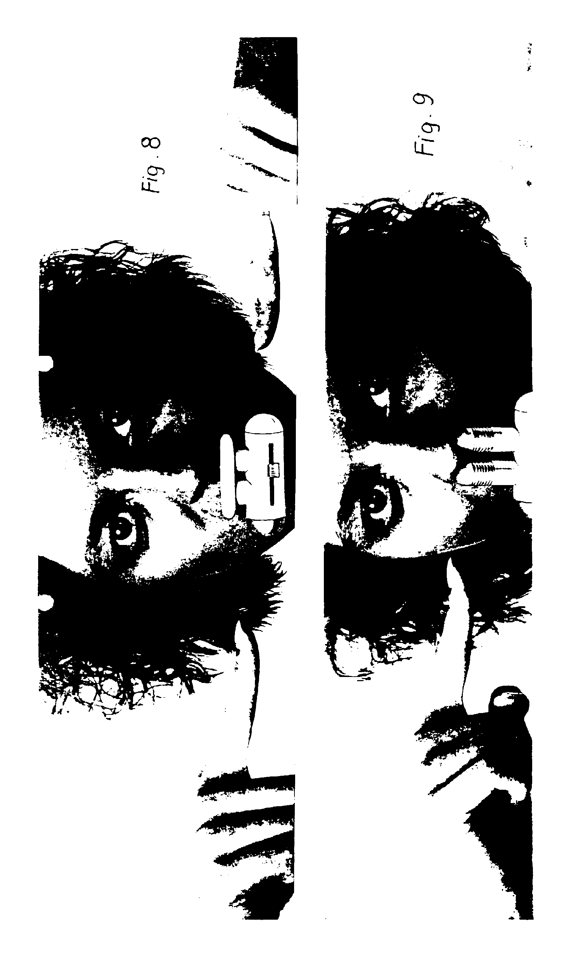

- Figur 8: das Gerät bei der Anwendung zum Inhalieren durch den Mund und
- Figur 9: das Gerät zum Inhalieren sowohl durch den Mund als auch durch die Nase.

Das Inhaliergerät besteht aus einem zylindrischen Mittelteil 1 mit halbkugelförmigen Endkappen 2 und 3. Das Mittelteil weist eine schlitzförmige öffnung 4 für den Eintritt von Frischluft in das Innere und eine mundseitige öffnung 5 für den Austritt der mit Wirkstoffen angereicherten Frischluft und für den Eintritt rückströmender, ausgeatmeter Luft auf. Die Mundöffnung 5 ist mit einem Anschlußstutzen 6 zum Aufschieben eines Mundstücks 7 aus einem hochelastischen Material versehen.

Im Innenraum des Mittelteils 1 ist ein Wirkstoffträger 8 angeordnet, der aus mehreren elastischen Armen 9 besteht, die unmittelbar mit der Endkappe 3 verbunden sind. Diese Arme halten einen Docht oder Tampon 10, angereichert mit ätherischen ölen oder anderen Wirkstoffen unter leichter Klemmwirkung. über den Wirkstoffträger ist eine hülsenförmige Dosierkappe 11 schiebbar, deren Boden 12 bis an das stirnseitige Ende 13 des Dochtes 10 schiebbar ist und den Wirkstoffträger hermetisch abdichtet, so daß weiterer Wirkstoff nicht verdunsten kann. Mit einem Handknopf 14 ist die Dosierhülse 11 in Längsrichtung des Mittelteils 1 verschiebbar. Dabei wird der Knopf 14 in der schlitzförmigen Öffnung 4 geführt.

Zusätzlich sind zwei Öffnungen zum Inhalieren durch die Nase vorgesehen. Die beiden Öffnungen sind mit zylindrischen Anschlüssen 15 zum Aufstecken von Nasenröhrchen 16 versehen.

Wünscht ein Patient die Inhalation lediglich durch den Mund, können die Nasenöffnungen mit Hilfe einer Abdeckplatte 17, an deren Unterseite sich zwei Schließkappen 18 befinden, verschlossen werden. Während der Inhalation kann also mit Wirkstoffen angereicherte Atemluft sowohl durch die Mundöffnung 5 als auch über die Nasenröhrchen 16 eingeatmet werden. Der von einer Stirnseite des Mittelteils in das Innere hineinkragende Wirkstoffträger wird dann durch seitliches Verschieben der Dosierhülse 11 mehr oder weniger freigegeben, um dadurch dosiert den Wirkstoff in der Atemluft verdunsten zu lassen.

## Patentansprüche

1. Gerät zum Inhalieren von Heilstoffen, gekennzeichnet durch ein Gehäuse (1, 2, 3) mit einem darin angeordneten Wirkstoffträger (8) und mindestens zwei ständigen Öffnungen (4, 5) für den Durchtritt von Luft, wobei eine der Öffnungen als Inhalieroder Mundöffnung (5) dient und mit einem Mundstück (6, 7) zum unmittelbaren Halten des Gerätes mit dem Mund ausgerüstet ist.

2. Gerät nach Anspruch 1, dadurch gekennzeichnet, daß der Wirkstoffträger (8) aus vier elastischen Armen besteht, die den mit Wirkstoff getränkten Docht oder Tampon unter leichter Vorspannung hälten.

3. Gerät nach Anspruch 2, dadurch gekennzeichnet, daß die Haltearme (9) unmittelbar an einer stirnseitigen Abschlußkappe (3) befestigt sind.

4. Gerät nach einem der Ansprüche 1 bis 3, dadurch gekennzeichnet, daß der Wirkstoffträger durch eine überschiebbare Dosierhülse (11) abdeckbar ist.

5. Gerät nach einem der Ansprüche 1 bis 4, dadurch gekennzeichnet, daß das Gehäuse zwei zusätzliche, mit aufsteckbaren Nasenröhrchen (16) versehene Inhalieröffnungen aufweist.

6. Gerät nach einem der Ansprüche 1 bis 5, dadurch gekennzeichnet, daß die Ansaugöffnung für Frischluft (4) schlitzförmig ausgebildet und zum Führen eines Betätigungsknopfes (14) der Dosierhülse (11) dient.

7. Gerät nach einem der Ansprüche 1 bis 6, dadurch gekennzeichnet, daß die Naseninhalieröffnungen bei Nichtgebrauch mit Hilfe von Verschlußkappen (18) verschließbar sind.

8. Gerät nach Anspruch 7, dadurch gekennzeichnet, daß die Verschlußkappen durch einen plattenförmigen Verbindungssteg (17) miteinander verbunden sind.

9. Gerät nach einem der Ansprüche 1 bis 7, dadurch gekennzeichnet, daß das Mundstück (6, 7) aus einem hochelastischen Kunststoff jeder Kieferform anpaßbar ist.
